## Europäisches Patentamt

## European Patent Office

## Office européen des brevets

(19)

(11) Veröffentlichungsnummer: **0 185 613**
**B1**

(12) **EUROPÄISCHE PATENTSCHRIFT**

(45) Veröffentlichungstag der Patentschrift: **16.01.91**

(51) Int. Cl.⁵: **A 61 K 6/02**

(21) Anmeldenummer: **85810532.3**

(22) Anmeldetag: **12.11.85**

(54) **Verfahren und Verwendung eines Isoliermittels zur Trennung eines in einer Kavität vorpolymerisierten Composite-Inlays.**

(30) Priorität: **05.12.84 CH 5779/84**

(43) Veröffentlichungstag der Anmeldung:
**25.06.86 Patentblatt 86/26**

(45) Bekanntmachung des Hinweises auf die
Patenterteilung:
**16.01.91 Patentblatt 91/03**

(84) Benannte Vertragsstaaten:
**DE FR GB IT NL SE**

(56) Entgegenhaltungen:
DE-A-2 756 256
DE-A-3 734 803
DE-C- 667 808
DE-C- 754 981

**M. Schug-Kösters et al.: Karies und Füllungsmethoden, Schriften zur Praxis des Zahnarztes, Band 4, Werk Verlag Dr. E. Banaschewski, München 1964, 121-130, 131 ff**

**Die Akte enthält technische Angaben, die nach dem Eingang der Anmeldung eingereicht wurden und die nicht in dieser Patentschrift enthalten sind.**

(73) Patentinhaber: **Coltène AG**
**Feldwiesenstrasse 20**
**CH-9450 Altstätten (SG) (CH)**

(72) Erfinder: **Schmid, Adalbert**
**Erlenstrasse 14**
**CH-9445 Rebstein (CH)**
Erfinder: **Kiener, Alfons**
**Wiesenstrasse 13**
**CH-9435 Heerbrugg (CH)**
Erfinder: **Kägi, Stephan, Dr.**
**Naglerstrasse 11a**
**CH-9443 Widnau (CH)**
Erfinder: **Claude, Jean Pierre**
**Kriessernstrasse 11a**
**CH-9450 Altstätten (CH)**

(74) Vertreter: **Frauenknecht, Alois J. et al**
**c/o PPS Polyvalent Patent Service AG,**
**Mellingerstrasse 1**
**CH-5400 Baden (CH)**

EP 0 185 613 B1

**EP 0 185 613 B1**

(56) Entgegenhaltungen:

I. Krejci et al., Universität of Zurich, Wear of MOD Composite Inlays, Unpublished Data Blankenau et al.: A direct Restorative Resin Inlay Technique, Quintessence International 5/1984, 515-6

H.G. Schaller et al.: Prüfung der Wandständigkeit verschiedner Komposite Kunststoffe im Seitenzahnbereich, DZZ 43, 914-8 (1988), Carl Hanser Verlag, München

D. Belz, Neue Materialien für Keramik und Kunststoff Inlays/Onlays, Dental Echo 8/87, 22-4

W. Drum, Guten Morgen Fräulein Neumann, Lehrbuch für die Zahnarzthelferin, Verlag Quintessenz Berlin, 1976, 126-7

P. Dionysopoulos & D.C. Watts, Dynamic mechanical properties of an inlay composite, J. Dentistry 17/3, 140-4 (1989)

Esthetic Dentistry Research Group Edited by M.B. Miller, Reality, 3, No.4, 190-197

Ingraham, Bassett, Koser, Der Goldguss, Quintessenz Verlag 1964

## Beschreibung

Die Erfindung betrifft ein Verfahren zur trennbaren Isolierung eines in einer Kavität vorpolymerisierten Composite-Inlays sowie die Verwendung eines Isoliermittels zur Durchführung des Verfahrens.

Composite-Füllungen werden heute in steigendem Masse als Zahnfüllmaterial eingesetzt und haben die Amalgame im Frontzahnbereich, wo vor allem ästhetische Betrachtungen eine Rolle spielen, vollständig verdrängt.

Im Seitenzahnbereich jedoch ist ihnen der eigentliche Durchbruch nicht gelungen, da sie bestimmte, für den klinischen Erfolg notwendige, Eigenschaften nicht aufweisen.

Dazu gehören insbesondere Porenfreiheit, Radioopazität, Abrasionsfestigkeit und vollständiger Randschluss. Es hat nicht an Versuchen gefehlt, diese Nachteile zu überwinden, um die unbestrittenen ästhetischen Vorteile und die Möglichkeit quecksilberfreier Füllungen für den Seitenzahnbereich nutzbar zu machen.

Die Problematik der Porenfreiheit wurde durch die Einführung von lichthärtenden Composites, welche kein manuelles Anmischen mehr benötigen, recht gut gelöst. Die Radioopazität konnte durch spezielle, mit Schwermetallen, wie Barium oder Strontium dotierte Glasfüller erreicht werden. Um den Verschleiss des Füllungsmaterials zu minimieren, wurden verschiedene Verbesserungen vorgeschlagen, welche zwar nicht exzellente, aber doch auch für den Seitenzahnbereich ausreichend gute Resultate brachten. Das Ausbrechen von Füllerteilchen aus der Harzmatrix, welches zu rauher Oberfläche und damit zu rascher Abrasion führt, wurde optimiert, indem die Füllstoffe mit speziellen Haftvermittlern (z.B. Silane) behandelt wurden. Durch dieses Verfahren konnte eine chemische Bindung zwischen Matrix und Füllstoff aufgebaut werden, welche wesentlich stärkeren Angriffskräften widerstehen kann. Zusätzlich dazu wurde auch die Teilchengrösse optimiert. Diese neuen sogenannten Hybridcomposites enthalten Füllstoffe mit einer mittleren Teilchengrösse im Bereich von etwa 0,5 bis 5 µm und einen Anteil an hochdisperser Kieselsäure. Solche Partikel könne weder weit aus der Füllungsoberfläche ragen noch beim Ausbrechen einen grossen Krater hinterlassen, welcher die ganze Füllung schwächt. Trotzdem weisen sie noch einen hohen Elastizitätsmodul und eine ausreichende Härte auf. Die eigentliche Problematik jedoch ist der vollständige Randschluss. Dessen Unversehrtheit ist notwendig, damit keine Mikroorganismen in den Randspalt eindringen und Sekundärkaries auslösen können.

Die Entstehung des Randspaltes liegt in der Polymerisationsschrumpfung begründet. Alle bekannten Composites weisen Schrumpfwerte im Bereich von 2,5 Vol.-% und grösser auf. Um trotzdem die Bildung eines Randspaltes zu vermeiden, wurde versucht, die Füllung mittels Haftstoffen an die Zahnhartsubstanz zu kleben. Dies ist allerdings bisher nur für kleine Füllungen mit Schrumpfwerten von wenigen Mikron gelungen, welche vollständig im Schmelzbereich liegen. Die Haftmittel für Dentin mögen die Schrumpfkräfte grosser Füllungen nicht auffangen und die Restaurationen werden unter normalen Mundtechniken schnell undicht. Mit verschiedenen Applikationstechniken wie Schichtaufbau und Schrumpfvektorumkehr (Aushärtung von der Zahnseite her) wurde zwar eine Verbesserung erreicht, welche aber für den klinischen Erfolg nicht ausreicht.

Ein neuer Ansatz zur Verbesserung der Randdichtigkeit wurde mit dem Composite-Inlay, ähnlich den in der Zahntechnik seit langem bekannten Gold- und Keramik-Inlays, gemacht. Der Zahnarzt nimmt zunächst einen Abdruck der präparierten Kavität mit einem der elastomeren Abformmaterialien. Darauf wird die Kavität provisorisch verschlossen und der Patient entlassen. Der Zahntechniker stellt nun aus dieser Abdruckform ein Modell aus Hartgips her. Das Hartgipsmodell wird in Modellsegmente zerteilt, und es erfolgt darauf die Modellation mit Composite. Es wird dabei ein lichthärtendes Material verwendet, dessen Polymerisation durch direkte Belichtung auf dem Modellstumpf erfolgt. Zur Erreichung besserer physikalischer Eigenschaften, insbesondere der Abrasionsresistenz und des Elastizitätsmodul, wird nach dem Entfernen des Inlays vom Stumpfmodell dieses in einem Ofen vergütet, z.B. 15 Minuten bei 100°C oder 5 bis 10 Minuten bei 120°C. Das entnommene Inlay wird ausgearbeitet und glanzpoliert und kann dem Patienten eingesetzt werden, nachdem der Zahnarzt das Provisorium entfernt und die Kavität gereinigt und getrocknet hat.

Das bekannte Verfahren erlaubt, dass erstens ein optimal geformtes und ausgehärtetes Material verwendet werden kann, dessen Polymerisation bereits vor dem Einpassen vollständig abgeschlossen ist und somit keinen Schwund mehr aufweist. Deshalb wird zusammen mit dem Zementiermittel auch ein optimaler Randschluss erreicht. Zudem können gute Kontaktpunkte, ideale Occlusionsgestaltungen und die Verhinderung von Überschüssen, insbesondere im approximalzervikalen Rand, erreicht werden. Nachteilig an diesem Verfahren ist, dass es sehr komplex und zeitaufwendig ist. Es benötigt mindestens zwei Zahnarztbesuche des Patienten und die Anfertigung eines Provisoriums. Zusätzlich besteht die Gefahr, dass sich auf dem langen Fertigungsweg ein Fehler einschleicht, welcher eine Wiederholung der Arbeit erfordert.

Diese Vorgehensweise kann nicht abgekürzt werden, da es bisher nicht gelungen ist, eine Nachbearbeitung eines vorpolymerisierten Inlays vorzunehmen, da dessen Entnahme ohne Zerstörung seiner Raumform nicht möglich war.

Aufgabe der Erfindung ist es daher, ein Verfahren und ein Isoliermittel der eingangs genannten Art zu schaffen, welche die direkte Legung eines Composite-Inlays ermöglichen, ohne für eine eventuelle Modellherstellung den Zahntechniker zu bemühen.

Diese Aufgabe wird erfindungsgemäss dadurch gelöst, dass in einer präparierten Kavität ein bei Körpertemperatur ein Gel bildendes Isoliermittel eingebracht wird, welches Gel als Trennmittel zwischen Kavität und dem anschliessend einzubringenden Compositematerial dient, dass das eingebrachte Compositematerial in einem oder mehreren Schritten appliziert und vorpolymerisiert wird, dass das so hergestellte Inlay herausgenommen, ausgehärtet und für die endgültige Legung in an sich bekannter Weise bearbeitet wird.

Das Isoliermittel zur Durchführung des Verfahrens besteht nach Anspruch 4 aus einer Zusammensetzung, welche bei Körpertemperatur ein Gel bildet, wobei zweckmässig nach Anspruch 5 das gelbildende Isoliermittel aus einer gebildenden Komponente und einem Alkohol gebildet ist.

Das Isoliermittel bildet nach dem Verdunsten des Alkohols einen dünnen Gelfilm, welcher das Verbinden des Composites mit dem Dentin der Kavität verhindert und somit seine spätere Entfernung ermöglicht.

Erst durch Verwendung des erfindungsgemässen Isoliermittels ist es in überraschender Weise gelungen, ein in einer Kavität vorpolymerisiertes Composite ohne Zerstörung seiner Feingestalt und Oberfläche und der Kavität zu entfernen und extern auszupolymerisieren und nachzubearbeiten. Die erfindungsgemäße Verwendung des Isoliermittels zur Herstellung der Trennschicht erfolgt somit als Applikationshilfe.

Das Verfahren dauert von der Fertigstellung der Kavität bis zur Politur des Zahnes weniger als 45 Minuten. Es hat den grossen Vorteil, dass das Inlay in einer einzigen Sitzung gelegt werden kann, während nach den bekannten Verfahren mehrere Tage erforderlich sind.

Die Durchführbarkeit des Verfahrens hängt von einer korrekten Inlaypräparation und insbesondere vom Isoliermittel ab, welches erst ermöglicht, das in der Kavität vorpolymerisierte Inlay zur weiteren Bearbeitung unbeschädigt aus der Kavität zu entnehmen.

Nach einer Weiterausbildung der Erfindung wird gemäss Anspruch 2 zur Kavität eine Matrize zur Formgebung des Inlays befestigt. Diese Matrize kann auch ausbombiert werden.

Vorteilhafterweise wird nach Anspruch 3 als Composite material ein lichthärtendes Hybridcomposite eingesetzt, wobei transparente Keile für die Fixierung der Matrize die teilweise Aushärtung erleichtern.

Gemäss dem Anspruch 5 besteht das Isoliermittel aus einer gelbildenden Komponente und einem wasserhaltigen Alkohol. Als Alkohol ist Äthylalkohol am besten geeignet, da er physiologisch unbedenklich ist. Es können aber auch andere hydrophile organische Lösungsmittel, wie beispielsweise andere kurzkettige Alkohole wie Methanol oder Isopropanol, Ketone wie Aceton, Methylethylketon, oder Aldehyde wie Ethanal oder Propanal, zur Herstellung des Isoliermittels verwendet werden.

Die gelbildende Komponente ist vorteilhaft nach Anspruch 6 ein organisches Quellmittel, z.B., wie es im Anspruch 7 als Agar-Agar angegeben ist.

Es ist dabei zweckmässig, die gelbildende Komponente zunächst mit dem hydrophilen Lösungsmittel anzuquellen und sie anschliessend in den vollständigen Gel zustand überzuführen.

Als organische Quellmittel sind Methylcellulose, Polysaccharide, Pektine, Agar-Agar oder Gelatine, allein oder gemischt, geeignet. Als besonders vorteilhaft hat sich das Quellmittel Agar-Agar erwiesen, welches in einer Menge von 0,5 bis 5 Gew.-%, vorzugsweise etwa 3 Gew.-%, in Alkohol-Wasser zur Anwendung kommt, wobei das Verhältnis Alkohol zu Wasser ca. 1:1 ist.

Die gelbildende Komponente kann gemäss Anspruch 8 zuch ein anorganisches Quellmittel sein. Dazu sind z.B. Aluminiumhydroxide, Kieselsäure und Montmorillonit geeignet. Als besonders vorteilhaft hat sich alkalifreies Aluminiumhydroxidgel erwiesen, wie es im Anspruch 9 angegeben ist.

Das Composite-Zahnfüllmaterial besteht vorzugsweise aus einem Hybridcomposite, bestehend aus 15 bis 30 Gew.-% einer Harzmatrix mit endständigen Methacrylgruppen und 70 bis 85 Gew.-% eines radioopaken Glasfüllers mit einer mittleren Teilchengrösse von 0,5 bis 5 µm, wobei 0 bis 10 Gew.-% des Glasfüllers durch hochdisperse Kieselsäure (HDK) ersetzt werden kann. Eine solche Composite-Zahnfüllung zeigt besonders hohe Abrasionsresistenz und lässt sich besonders leicht mittels des Isoliermittels aus der Kavität entnehmen. Es sind auch Zahnfüllungscomposites anderer Zusammensetzung verwendbar.

Die erfindungsgemäße Verwendung des Isoliermittels ist insbesondere zur direkten Legung von Inlays im Prämolaren- und Molaren-Bereich geeignet.

Die Arbeitsweise zum Legen eines Inlays mit dem erfindungsgemäss zu verwendenden Isoliermittel wird nachfolgend beispielsweise erläutert.

Nach der Präparation der Kavität des Zahnes und dem Glätten der rechtwinklig präparierten Ränder in bekannter Weise, wird eine passende Matrize zur Formgebung des Zahnes verkeilt und erforderlichenfalls ausbombiert. Zur Verkeilung können in vorteilhafter Weise transparente Keile (HAWE-NEOS DENTAL) verwendet werden, welche eine Lichtpolymerisation von der zervikalen Seite her erlauben. Nun wird das Isoliermittel in Form einer Flüssigkeit durch Sprühen oder mit einem Pinsel in die Kavität eingebracht. Auf die sich ausgebildete Isolierschicht wird ein geeignetes Composite in bekannter Weise mesial-occlusal appliziert und mit einem Kugelstopfer angestopft, und diese erste Portion währnd 1 Minute (mit Licht einer Wellenlänge im Bereich von 450 bis 500 nm und einer Stärke von ca. 70 mW) belichtet. Eine zweite Portion des Composites wird distalocclusal eingebracht und angestopft. Es folgt eine Grobformung der occlusalen Anatomie. Danach wird die zweite Portion unter gleichen Bedingungen auch eine Minute und anschliessend nochmals die ganze Füllung während ein bis zwei weiteren Minuten belichtet. Nach Entfernung der Matrize bleiben die Keile in Position. Es folgen die Grobausbauarbeiten der occlusalen-palatinalen-lingualen-buccalen Überschüsse. Das Herausheben des Inlays erfolgt mit einem Metallspatel. Eventuelle Störpunkte werden mit einem rotierenden Instrument entfernt. Das so vorpolymerisierte Inlay wird auf den Zahn zurückgesetzt und nach der Kontrolle entfernt. Zur vollständigen Aushärtung wird das Inlay während etwa 15 Minuten bei 100°C oder während etwa 5 bis 10 Minuten bei ca. 120°C im Heizofen, eventuell mit zusätzlicher Lichtaktivierung, vergütet. Nach dem Entfernen des nicht mehr benötigten Isoliermittels aus der Kavität, z.B. durch intensives Abspülen, werden die Schmelzränder geätzt. Vor der Zementierung werden die Innenflächen des Inlays mit einem Diamanten etwas aufgerauht, der Schmelz in bekannter Weise mit einer chemisch härtenden Harzlösung, bestehend aus einer Mischung von Bowen-Harzen mit additiven Stabilisatoren, Initiatoren und Aktivatoren, gegebenenfalls mit speziellen Haftverstärkern für Schmelz und/oder Dentin versetzt, beschichtet und das Inlay mit einem fliessfähigen Composite, vorzugsweise einem Hybridcomposite, in bekannter Weise einzementiert.

Diese Zementcomposites bestehen vorzugsweise aus einem langsam aushärtenden 2-Komponentenmaterial, welches für die optimale Randanpassung mit einem sehr feinteiligen Füller einer mittleren Teilchengrösse im Bereich von 0,2 bis 3 µm und einen Füllgrad von ca. 50 bis 75 Gew.-% aufweist. Für die visuelle und die röntgenographische Kontrolle empfiehlt sich eine opake und radioopake Konstruktion. Es ist wichtig, dass zum Einzementieren ebenfalls ein qualitativ hochwertiges Composite verwendet wird, um die Erosion des Zementspaltes zu vermeiden. Nach Entfernung interdentaler und occlusaler Überschüsse, beispielsweise mit Zahnseide, Strips, etc., ist das Inlay polierbar. Das ganze Verfahren dauert etwa 30 bis 45 Minuten.

Das erfindungsgemäße Isoliermittel wird nachfolgend anhand einiger Beispiele näher erläutert:

Beispiel 1

| | |
|---|---|
| Agar-Agar | 3,00 g |
| Wasser | 47,00 g |
| Aethanol (96%ig) | 50,00 g |
| | 100,00 g |

Das pulverförmige Agar-Agar wird zunächst 10 Minuten in Wasser gequollen, mit Aethanol versetzt und unter starkem Rühren zu einem kolloidalen Gel verteilt.

Beispiel 2

Je 10 g Celluloseäther der Typen MW 6'000, MK 20'000, CRW 5'000 der Firma WOLFF, WALSRODE AG, Deutschland, wurden in 500 ml 50 Vol.-% Alkohol-Wasser während 10 Minuten aufgekocht. Es bildet sich nach dem Abkühlen ein Gel, das eine ausgezeichnete Isolierwirkung aufweist.

Beispiel 3

80 g einer hydrophilen, hochdispersen Kieselsäure (HDK V 15, der Wacker Chemie, München) wurden mit 920 ml 50 Vol.-% Alkohol Wasser versetzt und während 10 Minuten unter Rückfluss gekocht. Es resultiert ein Gel mit ausreichender Isolierwirkung.

Beispiel 4

50 g Gelatine und 950 ml Alkohol: Wasser 1:1 werden 10 Minuten zum Sieden erhitzt. Nach dem Abkühlen resultiert ein Gel mit guter Isolierwirkung.

Beispiel 5

10 g Gelatine, 5 g Agar und 990 ml eines 50 Vol.-% Alkohol Wasser Gemisches werden zusammen während 10 Minuten unter Rückfluss erhitzt. Es resultiert ein standfestes Gel mit ausgezeichneter Isolierwirkung.

Durch das erfindungsgemässe Verfahren und das Isoliermittel ist es erstmalig möglich geworden, entgegen der Annahme der Fachwelt, ein Inlay direkt zu legen. Dadurch werden zahlreiche Arbeitsgänge überflüssig, wodurch auch eine Einsparung an kostspieligem Material erreicht wird.

**Patentansprüche**

1. Verfahren zur trennbaren Isolierung eines in einer Kavität vorpolymerisierten Composite-Inlays, dadurch gekennzeichnet, dass in eine präparierte Kavität ein bei Körpertemperatur ein Gel bildendes Isoliermittel eingebracht wird, welches Gel als Trennmittel zwischen Kavität und dem anschliessend einzubringenden Compositematerial dient, dass das eingebrachte Compositematerial in einem oder mehreren Schritten appliziert und vorpolymerisiert wird, dass das so hergestellte Inlay herausgenommen, ausgehärtet und für die endgültige Legung in an sich bekannter Weise bearbeitet wird.

2. Verfahren nach Anspruch 1, dadurch gekennzeichnet, dass gegebenenfalls zur Formgebung des Inlays eine Matrize verwendet wird.

3. Verfahren nach Anspruch 1, dadurch gekennzeichnet, dass als Compositematerial ein lichthärtendes Hybridcomposite eingesetzt wird.

4. Verwendung eines Isoliermittels zur Durchführung des Verfahrens nach Anspruch 1, das dadurch gekennzeichnet ist, dass es bei Körpertemperatur ein Gel bildet.

5. Verwendung eines Isoliermittels nach Anspruch 4, das dadurch gekennzeichnet ist, dass es eine gebildende Komponente und einen Wasserhaltigen Alkohol enthält.

6. Verwendung eines Isoliermittels nach Anspruch 5, das dadurch gekennzeichnet ist, dass die gelbildende Komponente ein organisches Quellmittel ist.

7. Verwendung eines Isoliermittels nach Anspruch 6, das dadurch gekennzeichnet ist, dass das organische Quellmittel Agar-Agar ist.

8. Verwendung eines Isoliermittels nach Anspruch 5, das dadurch gekennzeichnet ist, dass die gelbildende Komponente ein anorganisches Quellmittel ist.

9. Verwendung eines Isoliermittels nach Anspruch 8, das dadurch gekennzeichnet ist, dass das anorganische Quellmittel ein Aluminiumhydroxid ist.

## Revendications

1. Procédé pour isoler un inlay composite prépolymérisé dans une cavité de façon à pouvoir l'en retirer, caractérisé en ce qu'on introduit dans une cavité préparée un produit isolant formant gel à la température du corps, lequel gel sert ·d'agent de séparation entre la cavité et le matériau composite qu'on doit y introduire ensuite, en ce que le matériau composite introduit est appliqué en une ou plusieurs opérations et qu'il est prépolymérisé, et en ce qu'on retire l'inlay ainsi fabriqué, qu'on le laisse durcir et qu'on le travaille de façon connue pour la pose définitive.

2. Procédé selon la revendication 1, caractérisé en ce qu'on utilise éventuellement une matrice pour donner sa forme à l'inlay.

3. Procédé selon la revendication 1, caractérisé en ce qu'on utilise comme composite un composite hybride photodurcissable.

4. Utilisation d'un isolant pour mettre en oeuvre le procédé selon la revendication 1, qui est caractérisé par le fait qu'il forme un gel à la température du corps.

5. Utilisation d'un isolant selon la revendication 4, caractérisé en ce qu'il comporte un composant formant un gel et un alcool contenant de l'eau.

6. Utilisation d'un isolant selon la revendication 5, caractérisé en ce que le composant formant un gel est un agent gonflant organique.

7. Utilisation d'un isolant selon la revendication 5, caractérisé en ce que l'agent gonflant organique est l'agar-agar.

8. Utilisation d'un isolant selon la revendication 5, caractérisé en ce que le composant formant un gel est un agent gonflant inorganique.

9. Utilisation d'un isolant selon la revendication 8, caractérisé en ce que l'agent gonflant inorganique est un hydroxyde d'aluminium.

## Claims

1. Method for separable isolation of a composite inlay which is pre-polymerised in a cavity, characterised in that into a prepared cavity there is introduced an isolating agent which forms a gel at body temperature, which gel serves as a separating agent between the cavity and the composite material which is to be introduced subsequently, that the composite material introduced is applied in one or more steps and is pre-polymerised, that the inlay thus produced is removed, is hardened, and is processed for the final placing into position, in a manner known per se.

2. Method according to claim 1, characterised in that optionally a matrix is used for shaping the inlay.

3. Method according to claim 1, characterised in that a light-hardenable hybrid composite is used as the composite material.

4. Use of an isolating agent for carrying out the method according to claim 1, which is characterised in that it forms a gel at body temperature.

5. Use of an isolating agent according to claim 4, which is characterised in that it contains a gel-forming component and an aqueous alcohol.

6. Use of an isolating agent according to claim 5, which is characterised in that the gel-forming component is an organic swelling agent.

7. Use of an isolating agent according to claim 6, which is characterised in that the organic swelling agent is agar-agar.

8. Use of an isolating agent according to claim 5, which is characterised in that the gel-forming component is an inorganic swelling agent.

9. Use of an isolating agent according to claim 8, which is characterised in that the inorganic swelling agent is an aluminium hydroxide.